# FASCICULE DE BREVET EUROPEEN

(11) **EP 0 633 008 B1**
(45) Date de publication et mention de la délivrance du brevet: **20.05.1998**
(21) Numéro de dépôt: 93440053.2
(22) Date de dépôt: 06.07.1993
(51) Int. Cl.: A61F 7/00, F25D 3/10, F17C 9/02

(54) **Appareillage de cryothérapie**
Einrichtung für die Kryotherapie
Cryotherapy device

(43) Date de publication de la demande: 11.01.1995
(73) Titulaire: CRYONIC MEDICAL, 39110 Salins Les Bains (FR)
(72) Inventeur: Cluzeau, Christian, 67000 Strasbourg (FR)
(74) Mandataire: Puiroux, Guy

(56) Documents cités:
- EP-A- 0 256 730
- DE-A- 3 702 807
- DE-U- 8 519 992
- GB-A- 2 042 148

## Description

La présente invention concerne la cryothérapie, c'est-à-dire le traitement de diverses affections par l'action superficielle d'un froid intense provoquant un ensemble d'effets physiologiques tels que notamment :
- l'abaissement du seuil de la douleur
- la diminution de la spasticité
- un relâchement des fibres musculaires
- une action hémolytique
- une action anti-oedémateuse

Jusqu'à présent, cette technique faisait usage d'azote provenant de la gazéification instantanée d'azote liquéfiée à - 178 ° C, de sorte qu'elle était soumise à de nombreuses contraintes déterminant de nombreux inconvénients, ce qui en a largement limité l'application qui aurait pu trouver une extension à de nombreuses indications thérapeutiques.

Ainsi, l'azote liquéfié doit être conservé et mis en oeuvre dans un matériel lourd et encombrant, donc peu maniable, et duquel il s'évapore en permanence, ce qui entraine des pertes importantes, sa gazéification en vue de son application médicale impliquant cependant un réchauffage faisant intervenir un système électrique qui ne permet toutefois pas d'obtenir un froid immédiat, ni une pression élevée à la buse de sortie. Enfin, l'azote est relativement mal distribué géographiquement, et entraine des coûts d'exploitation élevés.

La présente intervention élimine tous ces inconvénients grâce à un nouvel appareillage faisant appel à l'anhydride carbonique liquéfié sous pression, gazéifié au point d'application situé à la sortie d'un système spécial d'éjection relié par une canalisation souple sous pression à un tube plongeur monté dans une bouteille d'alimentation en soi classique.

Un tel appareillage permet de disposer à tout moment d'un jet de CO2 à haute pression, de l'ordre de 50 bars, et à basse température, de l'ordre de - 78° C ; selon une caractéristique importante, il est prévu d'éjecter ce jet par un gicleur équipé d'un diffuseur permettant, par aspiration latérale d'air, de moduler à volonté la température et la taille du jet, tout en évitant la formation de carboglace.

A cet effet, l'appareillage selon l'invention comprend, en combinaison :
- Une bouteille d'anhydride carbonique liquéfié sous pression d'environ 50 bars, logée dans une carrosserie légère facilitant son transport et sa manipulation, et dans laquelle est disposé un tube plongeur ;
- Une canalisation résistante à la pression de 50 bars et raccordée d'un côté à l'extrémité du tube plongeur, et de l'autre côté à un système d'éjection avec gazéification ;
- Un système d'éjection se composant d'une soupape de commande de l'alimentation en CO2 liquide d'un gicleur, et d'un diffuseur destiné à la fois à empêcher la formation de carboglace à l'éjection du CO2 gazeux et à moduler la température et la dimension, c'est-à-dire la pression du jet éjecté.

Il convient de noter que l'utilisation du CO2 avait déjà été mentionnée en cryochirurgie ainsi qu'en pédiatrie, pour jouer un rôle d'anesthésiant local, mais que l'étendue de cette utilisation s'était trouvée limitée par deux facteurs : d'une part la formation de glace dans la tubulure d'éjection et d'autre part l'absence de moyens de modulation de la température et de la dimension du jet en fonction de la nature du traumatisme à traiter. Le document GB-A-2 042 148 décrit un système pour réfrigérer une chambre de congèlation avec un système de détente ne formant pratiquement pas de carboglace et avec des moyens pour moduler le jet d'anhydride carbonique.

L'invention représente un perfectionnement considérable à cette technique antérieure en éliminant ces deux facteurs. A cet effet, dans l'appareillage selon l'invention, le CO2 demeure liquide et sous pression jusqu'à la sortie du système d'éjection, grâce au raccordement de ce système au tube plongeur par une canalisation résistant à la pression de liquéfaction, tandis que ledit système d'éjection proprement dit se compose d'une part d'une soupape, équipée d'une manette de commande, et connectée à l'extrémité de ladite canalisation, et d'autre part d'une tubulure de sortie montée sur la sortie de cette soupape et à laquelle est adapté un diffuseur constitué par un tube concentrique, percé d'ouvertures d'admission d'air, la position de ce diffuseur déterminant la forme, la dimension et la température du jet de CO2 éjecté sur la zone du patient à traiter.

On va décrire l'invention plus en détail en se référant au dessin annexé, sur lequel :
- la figure 1 illustre schématiquement l'appareillage selon l'invention,
- la figure 2 est une vue latérale à plus grande échelle du système d'éjection du CO2,
- la figure 3 est une coupe axiale dudit système et
- la figure 4 illustre plus en détail le diffuseur équipant ce système.

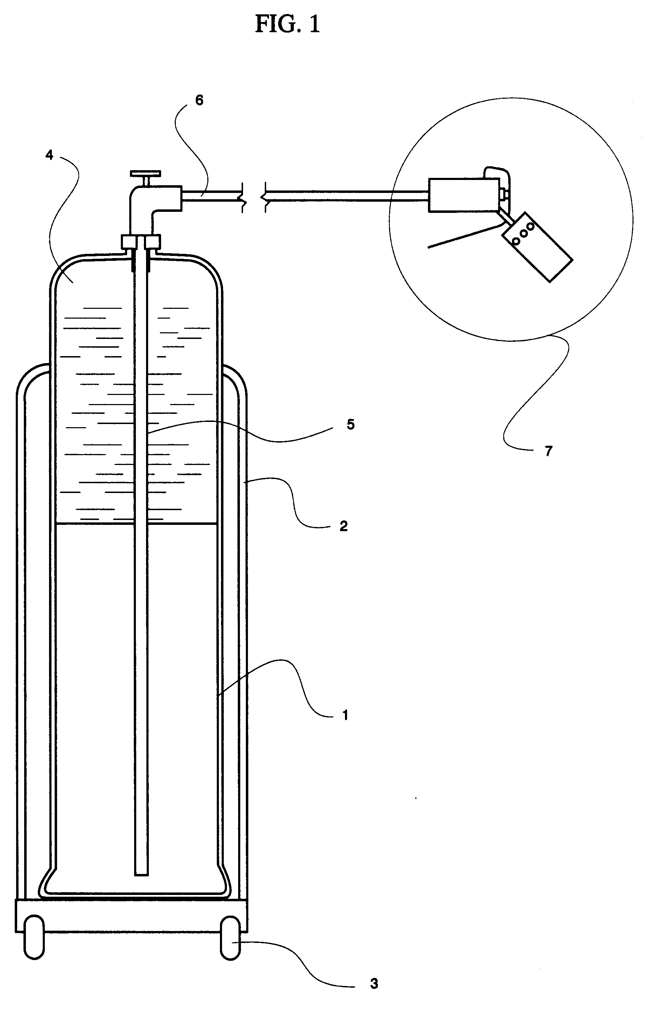

Sur la figure 1 de ce dessin, on a représenté schématiquement en 1 une bouteille classique d'anhydride carbonique liquéfié sous pression montée dans une carrosserie 2 destinée à en faciliter le déplacement et la manipulation, par exemple grâce à des roulettes 3.

Sur la tête de cette bouteille 1 est vissé un raccord 4, se prolongeant par un tube plongeur 5 et équipé d'une canalisation 6 capable de supporter la pression de liquéfaction du CO2, c'est-à-dire environ 50 bars, et raccordée au système d'éjection désigné par la référence générale 7.

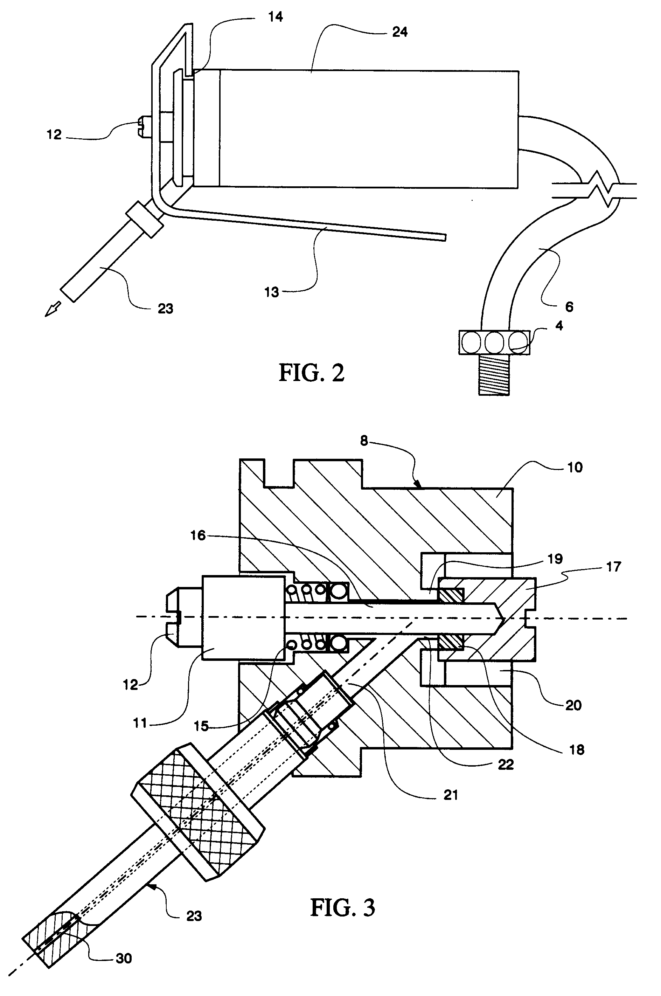

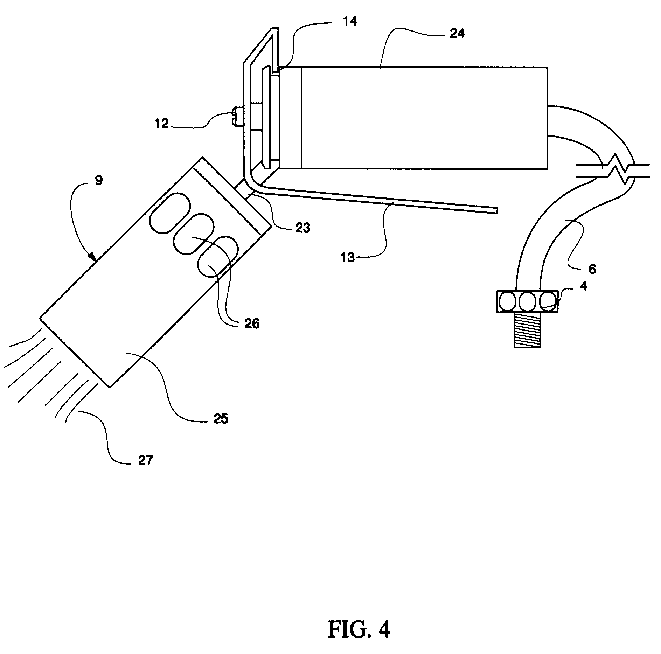

Comme l'illustrent les figures 2, 3 et 4, ledit système d'éjection se compose d'une part d'une soupape 8 (figure 3), et d'un diffuseur applicateur 9 (figure 4).

Comme on le voit sur la figure 3, la soupape 8 comporte un corps 10 et un plongeur axial 11 dont la tête 12 est soumise à une manette de commande 13 articulée en 14 sur le corps 10 en opposition à un ressort de compression 15, et dont la queue 16 porte la soupape proprement dite 17 dont la garniture d'étanchéité 18 est pressée contre le siège 19 du corps 10 par l'action du ressort 15. La canalisation 6 est connectée à la chambre amont 20 de la soupape, et la manoeuvre de la manette de commande 13 éloigne la soupape 17 de son siège 19, en mettant en communication la chambre amont 20 et la chambre aval 21 par un passage torique 22 créé entre la queue 16 et le corps 10. A cette chambre aval 21 est raccordée un tubulule de sortie 23 (figures 2, 3 et 4), par exemple en matière plastique. L'ensemble est également habillé par un capotage 24 facilitant une préhension ergonomique.

Selon une caractéristique importante de l'invention, la détente, qui provoque le passage du CO2 de la forme liquide à la forme solide, se produit dans une tubulure de très petit diamètre 30, tout à fait en aval de la chambre aval 21, dans laquelle la vitesse d'écoulement est très importante.

De ce fait, l'accumulation de carboglace est impossible dans cette tubulure 30, et la carboglace qui se forme est projetée à l'extérieur sous forme de particules micronisées qui se subliment instantanément, précisément dans la zone du patient à traiter.

A titre d'exemple, selon une réalisation préférentielle du système de détenteéjection 7 la chambre amont 20 alimentée en CO2 liquide par la canalisation 6 et la chambre aval 21 présentent un diamètre d'environ 5 mm, tandis que la tubulure 30 présente un diamètre de 0,5 mm. On peut donc considérer que la durée de séjour du CO2 dans la tubulure 23 est de quelques fractions de secondes, de sorte que la carboglace n'a pratiquement pas le temps de s'y former, et que, pour ce qui est des quelques particules qui peuvent se former à la sortie, elles sont de l'ordre du 1/10 mm et sont balayées vers l'extérieur où elles se subliment.

Selon une autre caractéristique importante, à la tubulure de sortie 23 est adapté un manchon 25 percé à sa base d'orifices 26 ayant pour effet d'aspirer l'air ambiant par entrainement du type Venturi créé par le jet de CO2 gazeux éjecté vers l'extérieur dans la région 27. Selon le nombre d'orifices 26 et les dimensions du manchon 25, il est donc possible de moduler à la fois la quantité d'air entrainé et par suite la température et la pression régnant dans la région 27 où sera placée la zone du patient à traiter, et la dimension de cette zone en fonction de sa distance par rapport au système 7.

Il convient également de noter qu'à cet appareillage est intégré un dispositif additionnel permettant de palper en permanence la température de la peau du patient dans la zone traitée et dont les mesures sont indiquées visuellement et/ou par un système d'avertissement sonore, de sorte que le praticien a la possibilité de procéder à tout moment à ladite modulation en fonction des besoins, et en particulier ne court pas le risque de prolonger le traitement au point que la peau voie sa température descendre jusqu'à 0° C.

Ainsi, grâce à l'appareil selon l'invention, il devient possible d'étendre l'application de la cryothérapie gazeuse à de très nombreuses indications, parmi lesquelles on peut citer, à titre d'exemples :
- En traumatologie, le traitement de la douleur aigue provoquée par exemple par des entorses, luxations, hématomes, oedèmes, claquages et déchirures musculaires,
- En rhumatologie, le traitement de tout phénomène inflammatoire douloureux, provoqué par exemple par des tendinites, algodystrophies, périarthrites et polyarthrites inflammatoires,
- En neurologie, le traitement de la spasticité,
- En hématologie, le traitement des conséquences de l'hémophilie, ainsi que bien d'autres traitements.

## Revendications

1. Appareillage pour la cryothérapie en phase gazeuse, qui comprend, en combinaison :
- une bouteille (1) d'anhydride carbonique liquéfié sous pression, montée dans une carrosserie (2) facilitant son déplacement et sa manipulation, sur la tête de cette bouteille étant vissé un raccord (4) se prolongeant par un tube plongeur (5) s'étendant sur pratiquement toute la longueur de la bouteille,
- une canalisation souple (6) résistant à la pression de liquéfaction de l'anhydride carbonique et connectée à une extrémité audit raccord et à l'autre à un système de détente et d'éjection (7) de l'anhydride carbonique gazéifié
- ledit système de détente et d'éjection se composant d'une soupape actionnée par une manette (13) de commande extérieure, une chambre (21) située en aval par rapport à cette soupape se prolongeant par une tubulure de sortie (23) présentant une section à faible diamètre (30) de telle manière qu'en raison de la vitesse propre du gaz qui la traverse en s'y détendant, il ne s'y forme pratiquement pas de carboglace,
- ladite tubulure de sortie étant équipée de moyens (25) pour moduler la température, la pression et les dimensions du jet d'anhydride carbonique gazeux projeté sur la zone du patient à traiter.

2. Appareillage selon la revendication 1, caractérisé en ce que lesdits moyens consistent en un manchon (25) concentrique à ladite tubulure, et présentant à son extrémité amont des orifices (26) pour l'aspiration d'air par effet Venturi, la quantité de cet air aspiré, et par suite la température et la pression régnant dans ladite zone, dépendant du nombre de ces orifices, et les dimensions dudit jet dépendant du diamètre dudit manchon.

3. Appareillage selon les revendications 1 et 2, caractérisé en ce que, additionnellement, il comporte des moyens pour palper en permanence la température de la peau du patient dans la zone traitée, et avertir le praticien de cette température.

## Claims

1. Equipment for gas-phase cryotherapy, which comprises, in combination:
- a bottle (1) of carbon dioxide liquefied under pressure, mounted on a trolley (2) to facilitate its movement and handling, with a connector (4) screwed to the head of the said bottle, the said connector ending in an immersion tube (5) which extends down into and along almost the entire length of the bottle,
- a flexible tube (6) which can withstand the liquefaction pressure of carbon dioxide and which is connected at one end to the said connector and at the other end to a pressure-drop and outlet system (7) for the gaseous carbon dioxide,
- the said pressure-drop and outlet system consisting of a valve activated by an external control hand-lever (13), a chamber (21) situated downstream from this valve and extending into a outlet tube (23) with a cross-section of small diameter (30) such that by virtue of the actual velocity of the gas passing through it while expanding almost no dry ice is formed,
- the said outlet tube being fitted with means (25) to modulate the temperature, the pressure and the size of the gaseous carbon dioxide jet projected onto the area of the patient to be treated.

2. Equipment according to Claim 1,
**characterized in that**
the said means consist of a sleeve (25) concentric with the said tube, and having at its upstream end orifices (26) to draw in air by the Venturi effect, the quantity of this air drawn in and consequently the temperature and pressure prevailing in the said area being dependent on the number of such orifices, and the size of the said jet depending on the diameter of the said sleeve.

3. Equipment according to Claims 1 and 2,
**characterized in that**
in addition, it comprises means to sense continuously the temperature of the patient's skin in the area treated, and to alert the practitioner to that temperature.

## Patentansprüche

1. Einrichtung für die Kryotherapie in Gasphase, die in Kombination umfasst:
- eine Flasche (1) mit verflüssigtem Kohlendioxid unter Druck, die in einem Gestell (2) angebracht ist, welches ihre Fortbewegung und ihre Handhabung erleichtert, wobei auf den Hals dieser Flasche ein Verbinder (4) aufgeschraubt ist, der durch ein Tauchrohr (5) verlängert ist, das sich über praktisch die gesamte Länge der Flasche erstreckt,
- eine flexible Leitung (6), die den Verflüssigungsdruck des Kohlendioxids aushält und an einem Ende an den Verbinder und am anderen an ein System (7) zur Entspannung und zum Ausstoßen des vergasten Kohlendioxids angeschlossen ist,
- wobei das besagte System zur Entspannung und zum Ausstoßen aus einem durch einen äußeren Bedienungshebel (13) betätigten Ventil besteht, wobei eine in Bezug zu diesem Ventil stromabwärtige Kammer (21) durch ein Austrittsrohr (23) verlängert ist, das einen Querschnitt mit geringem Durchmesser (30) aufweist, so daß aufgrund der Eigengeschwindigkeit des Gases, welches es unter Entspannung durchströmt, sich dort praktisch kein Kohlendioxideis bildet,
- wobei das besagte Austrittsrohr mit Mitteln (25) zum Verändern der Temperatur, des Drucks und der Abmessungen des auf die zu behandelnde Zone des Patienten gerichteten gasförmigen Kohlendioxidstrahls versehen ist.

2. Einrichtung nach Anspruch 1, dadurch gekennzeichnet, daß die besagten Mittel aus einer zu dem besagten Rohr konzentrischen Muffe (25) bestehen und an ihrem stromaufwärtigen Ende Öffnungen (26) zum Ansaugen von Luft durch den Venturi-Effekt aufweisen, wobei die Menge dieser angesaugten Luft, und folglich die Temperatur und der Druck, die in der besagten Zone herrschen, von der Anzahl dieser Öffnungen abhängen, und die Abmessungen des besagten Strahls vom Durchmesser der besagten Muffe abhängen.

3. Einrichtung nach Anspruch 1 und 2, dadurch gekennzeichnet, daß sie zusätzlich Mittel aufweist, um die Temperatur der Haut des Patienten in der behandelten Zone dauernd abzutasten und dem Arzt diese Temperatur zu melden.
